# FASCICULE DE BREVET EUROPEEN

(11) **EP 0 528 701 B1**
(45) Date de publication et mention de la délivrance du brevet: **15.10.1997**
(21) Numéro de dépôt: 92401889.8
(22) Date de dépôt: 02.07.1992
(51) Int. Cl.: C12N 9/74, C12N 9/96, A61K 38/48, A61K 35/16, A61L 25/00

(54) **Procédé de préparation d'un concentré de thrombine humaine destiné à un usage thérapeutique**
Verfahren zur Herstellung eines menschlichen Thrombin-Konzentrats für therapeutische Verwendung
Process of preparation of a human thrombin concentrate for therapeutic use

(30) Priorité: 18.07.1991 FR 9109075
(43) Date de publication de la demande: 24.02.1993
(73) Titulaire: ASSOCIATION POUR L'ESSOR DE LA TRANSFUSION SANGUINE DANS LA REGION DU NORD, 59000 Lille (FR)
(72) Inventeur: Michalski, Catherine, F-59800 Lille (FR); Dernis, Dominique, F-59529 Marquette lez Lille (FR)
(74) Mandataire: Lepeudry-Gautherat, Thérèse

(56) Documents cités:
- EP-A- 0 378 798
- EP-A- 0 439 156
- EP-A- 0 505 604
- WO-A-83/03760

## Description

La présente invention concerne un procédé de préparation d'un concentré de thrombine humaine destiné à un usage thérapeutique, purifié à l'échelle industrielle à partir d'une fraction du plasma humain.

La thrombine est une sérine-protéase générée dans le sang circulant par l'activation de son précurseur inactif, la prothrombine. Son rôle est fondamental dans le processus de la coagulation : elle coupe le fibrinogène en monomères de fibrine et elle active, par coupure protéolytique, le Facteur XIII qui stabilise le réseau de fibrine.

Elle intervient aussi dans d'autres processus physiologiques : elle initie les réactions de sécrétion et d'agrégation plaquettaire, facilitant ainsi la formation du clou hémostatique. Elle active aussi les protéines du système Complément. Elle a un effet mitogène sur les fibroblastes ce qui accélère la cicatrisation des vaisseaux sanguins lésés.

Par ces diverses propriétés, la thrombine trouve des applications thérapeutiques en tant qu'agent hémostatique local. Actuellement, dans ces applications cliniques on utilise de la thrombine animale, équine ou bovine. Ces préparations ne sont pas toujours parfaitement purifiées et leur application peut être à l'origine de réactions immunologiques dues à la surcharge en protéines hétérologues. L'usage de thrombine bovine comporte en outre le risque de transmission de maladies infectieuses récemment diagnostiquées et encore mal maîtrisées comme l'encéphalite bovine spongiforme (ou maladie des vaches folles).

La purification de la thrombine, comme celle de nombreuses enzymes, pose des problèmes pour maintenir la totalité de son activité enzymatique. En effet, l'α-thrombine native est généralement instable et, par autolyse ou par protéolyse limitée, elle donne des dérivés β- et γ-thrombines qui ont perdu une grande partie de leur activité coagulante sur le fibrinogène.

Les techniques de purification connues s'appliquent surtout à la thrombine bovine : elles comprennent la chromatographie sur résines échangeuses d'ions comme la DEAE-cellulose (Yin et al. J. Biol. Chem., 1968, 243, 112) éventuellement associée à une filtration sur Séphadex®- G 100 (Baughman et al. J. Biol.Chem., 1967, 242, 5252-5259) ; certaines méthodes permettent de séparer les formes β et γ sur colonne de phosphocellulose (Rosenberg et al., 1970, 245, 5049) et par association de plusieurs chromatographies d'échanges d'ions (Batt et al. J. Biol. Chem. 1970, 245, 4857).

De nouvelles résines ont également été utilisées comme le sulphoéthyl ou sulphopropyl Séphadex® (Lundlad-Biochemistry, 1971, 10, 2501) ainsi que des chromatographies d'affinité sur des supports comme l'héparine-Sépharose® (Nordeman et al. Thromb. Res., 1977, 11, 799-888) et p-aminobenzamidine-agarose (Hixson et al. Arch. Biochem. Biophys., 1973, 154-501).

Deux publications décrivent la purification de thrombine humaine, l'une sur benzamidine-sphérodex® (Lorne et al. Rev. Fr. Transf. Hémobiol., 1989, 32, 391-403) avec un rendement assez faible (11 à 20 U-NIH/nl), l'autre sur de nouveaux polystyrènes (Fischer et al. J. Chromatography, 1986, 363, 95-100) mais ce produit contient du facteur V bovin (voir plus bas).

Pour obtenir de la thrombine, il faut disposer non seulement d'une source de son précurseur, la prothrombine, mais également, en fonction du mode d'activation choisi, d'une concentration suffisante des autres facteurs de la coagulation impliqués dans le processus d'activation. Différentes méthodes on été décrites permettant d'activer la prothrombine en thrombine . Fenton et al. (Biochim. Biophys. Acta, 1971, 229, 26-32 et J. Biol. Chem., 1977, 252, 3587-3598) ont décrit la préparation de thrombine à partir de la fraction III de Cohn extraite sur résine, par addition de chlorure de calcium et de thromboplastine tissulaire extraite de cerveau humain. La réaction peut être accélérée par l'addition de Facteur V d'origine bovine (Bernamon-Djiane-Coagulation, 1968, 1, 259).

On peut aussi utiliser des activateurs spécifiques extraits de venins de serpents (Gosh et al. Thromb. Res., 1980, 20, 281).

Deux procédés de préparation de thrombine humaine à usage thérapeutique ont aussi été décrits :
- dans la demande de brevet EP 0 378 798 le procédé comprend une adsorption du plasma sur une matrice constituée de préférence de DEAE-Fractogel®, l'activation et l'élution de la thrombine et éventuellement une purification supplémentaire sur Fractogel®-sulfate. La thrombine obtenue a une très haute activité spécifique mais le rendement est assez faible ;
- dans la demande de brevet EP 0 443 724 cité en vertu de l'article 54(3) et (4) CBE le procédé comprend une adsorption du plasma sur échangeur d'ions à base d'agarose, l'activation de la thrombine par addition de CaCl₂, de phospholipides et de thromboplastine de cerveau de lapin, et une purification par chromatographie sur sulfopropyl-Sepharose®.

Les différentes méthodes décrites présentent divers inconvénients majeurs lorsqu'on envisage la préparation de très grands volumes de thrombine humaine destinée à un usage thérapeutique. Ainsi, la thromboplastine de cerveau humain ou animal est difficile à obtenir et constitue un facteur limitant pour traiter plusieurs centaines de litres de plasma.

L'activation au venin de vipère est difficilement compatible avec l'usage chez l'homme. L'utilisation de Facteur V bovin s'est révélée particulièrement dangereuse parce que les quantités résiduelles qu'on peut injecter aux patients sont à l'origine de graves réactions immunologiques.

C'est pourquoi la Demanderesse a développé un procédé permettant de préparer de la thrombine humaine purifiée et concentrée, ne comprenant aucune addition de matériel hétérologue et ayant, de plus, subi un traitement d'inactivation virale. Le procédé est simple et compatible avec une application à de grands volumes, à l'échelle industrielle.

Le matériel de départ est la fraction PPSB du plasma humain (PPSB : proconvertine ou F VII, prothrombine, facteur de Stewart ou F X et facteur antihémophilique B ou F IX), ainsi, toutes les molécules utiles à l'activation de la prothrombine sont présentes dans le mélange initial : les Facteurs VII, IX, X, les phospholipides et les cofacteurs V et VIII.

Le procédé mis en oeuvre par la Demanderesse comprend la succession des 6 étapes suivantes :
a) La fraction de PPSB qui est un dérivé de surnageant de plasma cryoprécipité est adsorbée sur DEAE-Sephadex® et lavée en tampon citrate à pH7 contenant du chlorure de sodium de 0,20 M à O,23 M et de préférence 0,23 M, ce qui permet d'éliminer le fibrinogène et les traces d'inhibiteurs qui freineraient la réaction suivante d'activation de la thrombine. Le PPSB est ensuite élué par augmentation de la concentration de chlorure de sodium à O,50 M.
b) L'activation de la prothrombine en thrombine est amorcée par l'addition de chlorure de calcium à une concentration finale comprise entre 5 et 10 mM et de préférence de 7mM. (On a observé qu'un excès de CaCl₂ inhibait la réaction d'activation). Cette étape comprend une première incubation de courte durée suivie d'une seconde, plus longue et mise en oeuvre à une température inférieure.
   Ainsi, e mélange est incubé, dans un premier temps, à 37°C pendant 2 heures. Ce traitement permet déjà la génération de 300 à 350 U NIH de thrombine par ml de PPSB (U NIH : unités définies par le National Institute of Health - USA). On a constaté qu'une incubation plus longue à cette température n'améliorait pas le résultat.
   Dans un deuxième temps, l'incubation est poursuivie à 24°C pendant au moins 16 heures, ce qui permet d'obtenir de 700 à 1000 U NIH de thrombine/ml de PPSB.
c) Le PPSB ainsi recalcifié est soumis, tel quel, à un traitement d'inactivation virale par solvant-détergent et plus particulièrement par l'addition de TnBP 0,3 %/Tween® 1 % ; l'incubation est poursuivie à 24°C pendant au moins 6 heures et, d'une manière générale pendant une nuit. Il faut noter qu'il est important d'effectuer ce traitement après l'activation de la thrombine parce que, si on l'effectue avant, c'est-à-dire directement sur le PPSB, on élimine des facteurs tels que les phospholipides qui sont nécessaires à la réaction d'activation.
d) La purification de la thrombine est ensuite effectuée par une seule étape de chromatographie d'échange d'ions, et plus particulièrement sur un échangeur fort de cations. On utilise préférentiellement un gel rigide constitué d'une matrice d'agarose substituée par des groupements CH₂ - SO₃, par exemple la S-Sépharose® FF (Pharmacia). La chromatographie est effectuée en milieu comprenant du gluconate de sodium 40mM et du chlorure de sodium 0,01 M, à pH 6,5. Le gluconate de sodium a un effet protecteur tout à fait favorable sur l'activité biologique de la thrombine.
   Après adsorption de la thrombine sur la colonne, celle-ci est lavée en milieu gluconate de sodium 150 mM, NaCl 0,04 M à pH 6,5.
   Les conditions d'élution de la thrombine ont été ajustées pour obtenir un pic chromatographique étroit, et plus particulièrement par une augmentation de la concentration de chlorure de sodium à 0,2 M et de gluconate à 150 mM.
   L'utilisation d'un milieu à base de gluconate de sodium présente, en outre, l'avantage d'éviter l'étape de dialyse qui est indispensable avec les tampons phosphate classiques.
e) Dès son élution de la colonne de chromatographie, la thrombine en solution dans le tampon gluconate est additionnée d'un mélange de stabilisants comprenant 2 g/l d'albumine, 5 g/l de saccharose et du CaCl₂ 60 mM, le rôle de ces stabilisants étant particulièrement important dans l'étape suivante.
f) La préparation de thrombine est ensuite concentrée, de préférence par ultrafiltration, conditionnée en volumes adaptés à l'utilisation thérapeutique ultérieure et lyophilisée.

La présence d'albumine comme stabilisant s'est révélée indispensable au cours de l'étape de concentration.

On a constaté que le saccharose, de préférence à tous les autres sucres et acides aminés qui ont été essayés, joue un rôle stabilisant très significatif sur le concentré à l'état liquide, au cours de l'étape de conditionnement qui peut être fort longue quand on répartit la solution en volumes de 1 à 5 ml.

La présence de CaCl₂ et de saccharose s'est révélée essentielle comme stabilisant en cours de lyophilisation.

Le procédé selon la présente invention présente plusieurs particularités qui le distinguent nettement des autres procédés décrits (plus particulièrement Lorne et al. et Fischer et al. cités plus haut) et qui procurent au produit final ses avantage remarquables de pureté et de très haute activité spécifique.

En résumé, ces caractéristiques sont :
- l'utilisation d'une fraction PPSB préalablement lavée et débarrassée du fibrinogène et d'inhibiteurs ;
- le choix des conditions de recalcification : concentration en chlorure de calcium et deux températures succesives d'incubation ;
- le traitement d'inactivation virale par solvant-détergent effectué entre les étapes d'activation et de purification de la thrombine et non sur le PPSB, comme cela semblerait souhaitable pour des raisons de sécurité de manipulation, mais qui, à ce stade, dénature les phospholipides qui sont nécessaires à la réaction d'activation de la thrombine ;
- la chromatographie sur un gel échangeur fort de cations et l'utilisation, pour cette étape, d'un milieu protecteur spécifique à base de gluconate de sodium ;
- le choix d'un mélange de stabilisants du produit fini qui protège son activité pendant les 3 dernières manipulations de concentration, distribution et lyophilisation.

L'invention a en conséquence pour objet un procédé de préparation d'un concentré de thrombine humaine à usage thérapeutique tel que revendiqué dans la revendication 1 comprenant une purification par voie chromatographique d'une fraction PPSB du plasma préalablement recalcifié par addition de CaCl₂ comprenant plus particulièrement un traitement de la fraction PPSB de départ pour éliminer le fibrinogène et les inhibiteurs d'activation de la thrombine, par lavage au chlorure de sodium, et, ensuite, une purification par voie chromatographique sur un gel échangeur fort de cations, en présence de gluconate de sodium.

Selon une autre caractéristique de l'invention, le procédé comprend un traitement d'inactivation virale par solvant-détergent qui est effectué après la recalcification de la fraction de PPSB lavée et avant la purification de la thrombine activée par chromatographie.

Selon une autre caractéristique de l'invention, le procédé comprend l'addition, dans la solution de thrombine éluée de la chromatographie, d'un mélange de stabilisants comprenant de l'albumine, du saccharose et du chlorure de calcium.

Le procédé selon l'invention est particulièrement remarquable parce qu'il s'adapte à de très grands volumes de matériau de départ et, ainsi, permet de produire, en un seul lot, jusqu'à 16 millions d'unités NIH de thrombine (soit 150 fois plus que par d'autres procédés décrits, comme dans la demande de brevet EP-O 378 798) et cela, sans interférer avec la purification des autres dérivés sanguins d'une plus grande importance économique (comme les facteurs de la coagulation sanguine, l'albumine, les immunoglobulines etc.)

La présente demande révèle un concentré de thrombine obtenu par le procédé décrit et qui montre une activité coagulante au moins égale à 500 U NIH/ml et une activité spécifique au moins égale à 1000 U NIH/mg de protéines.

Sa concentration élevée et son activité spécifique distinguent ce concentré d'un autre produit préparé selon un autre procédé (Lorne et al. cité plus haut) et qui n'atteint qu'une activité de 11 à 20 U NIH/ml.

De plus, ce concentré étant lyophilisé, son activité coagulante, au cours du temps, est d'une stabilité particulièrement remarquable.

Ce concentré de thrombine est donc parfaitement bien adapté à une application thérapeutique chez l'homme.

Il peut être utilisé tel quel, comme agent hémostatique local.

Il peut également être utilisé pour former de la colle biologique par addition à un concentré de fibrinogène comprenant de faibles quantités de Facteur XIII et de fibronectine, tel que décrit par la Demanderesse dans la demande de brevet EP-0 305 243. Le mode de formation de la colle biologique reproduit la dernière phase de la coagulation, c'est-à-dire la formation du réseau de fibrine. La thrombine coupe la molécule de fibrinogène en monomères de fibrine. Elle active le facteur XIII en présence des ions calcium, qui vient sous sa forme activée stabiliser la fibrine soluble en un caillot insoluble, ferme et non friable. Le pouvoir hémostatique du produit permet ainsi de limiter les saignements opératoires et postopératoires et de renforcer l'hémostase locale chez les patients présentant un déficit constitutionnel ou acquis de la coagulation (en particulier les malades sous anticoagulant).

Les exemples suivants illustrent l'invention sans en limiter la portée.

### Exemple 1

### a) - Préparation de la fraction PPSB.

Le matériel de départ est un surnageant de cryoprécipité de plasma que l'on obtient par décongélation et centrifugation à 0 - 3°C.

1000 à 1500 litres de surnageant sont adsorbés sur DEAE-Sephadex® A50 en présence de sérum physiologique, à raison de 1,5 g de résine par litre de surnageant. Après lavage au chlorure de sodium 0,23 M dans un tampon citrate 0,01 M à pH 7, le PPSB est élué par du chlorure de sodium 0,50 M dans le même tampon, concentré à 40 g/l de protéines, et additionné de lysine 2,5 g/l. Il est ensuite ajusté à une osmolarité d'environ 290 mosm/l à pH 7,0.

Le PPSB ainsi préparé contient, outre la prothrombine à la concentration d'environ 50 U/ml, les facteurs de coagulation X, IX et VII aux concentrations respectives d'environ 45 U/ml, 45 U/ml et 5 U/ml. Il contient également 5 à 6 U/ml des facteurs Vc et VIIIc ainsi que des phospholipides dont la présence est mise en évidence par le temps de génération de thrombine (ou TGT 50).

Le lavage par le chlorure de sodium permet d'éliminer le fibrinogène ainsi que certains inhibiteurs qui, lorsqu'ils sont présents dans le PPSB, freinent la réaction de recalcification.

### b) - Conditions de recalcification.

9 à 11 litres de PPSB fraîchement préparés ou après décongélation sont filtrés stérilement puis, additionnés de 7 ml de CaCl₂ 1M par litre de PPSB, en bécher ou cuve inoxydable stérile de 30 litres et placés au bain-marie à 37°C, pendant 2 heures. Après cette première étape on obtient environ 300 U NIH de thrombine/ml.

La température est ensuite abaissée à 24°C et le mélange est maintenu à cette température pendant encore 16 heures. Le taux de thrombine obtenu en fin de traitement est de 700 à 1000 unités NIH/ml de PPSB.

L'activité spécifique de la thrombine, à ce stade, est de 20 à 40 U NIH/mg de protéines.

### c) - Inactivation virale.

L'inactivation des contaminants viraux éventuels s'effectue par addition de TnBP à 0,3 % et Tween® 80 à 1 % en concentration finale, à la température de 24°C, durant au moins 6 heures. En pratique, le mélange est laissé une nuit supplémentaire en milieu TnBP/Tween® 80.

On n'observe pas de perte de l'activité thrombinique pendant cette étape. L'activité spécifique de la thrombine en fin de traitement TnBP/Tween® 80 est de 20 à 40 U NIH/mg de protéines.

Le mélange est ensuite congelé à -30°C, en poches de plastique, par aliquotes de 1 à 3 litres.

### d) - Purification de la thrombine.

Cette étape a pour but de séparer la thrombine du mélange réactionnel contenant le TnBP/Tween® 80, les facteurs activés de la coagulation et toutes les autres protéines du PPSB, pouvant être partiellement dégradées par les sérine-protéases.

On effectue une chromatographie sur un gel échangeur fort de cations : la S-Sepharose® FF (Pharmacia) qui est un gel rigide constitué d'une matrice d'agarose substitué par des groupements CH₂-SO₃.

La thrombine qui est peu stable, est protégée pendant le déroulement de la chromatographie par l'utilisation de gluconate de sodium à pH 6,5, dans les conditions suivantes : après décongélation, 10 à 12 litres de PPSB/thrombine/résidus de solvant-détergent sont ajustés à pH 6,5 puis injectés sur une colonne d'environ 16 litres de S-Sepharose® FF équilibrée en milieu constitué de gluconate de sodium 40 mM et de NaCl 0,01 M, à pH 6,5. La majorité des protéines du PPSB ainsi que le TnBP et le Tween® 80 ne sont pas retenus par la colonne. Le gel est ensuite soumis à un lavage en milieu gluconate de sodium 150 mM, NaCl 0,04 M, à pH 6,5. La thrombine est ensuite éluée par l'addition de chlorure de sodium 0,20 M dans le même milieu.

Dès son élut ion de la colonne, la thrombine est recueillie en présence des stabilisants suivants : albumine 2 g/l, saccharose 5 g/l et chlorure de calcium 60 mM. Le produit est ensuite concentré par ultrafiltration, ajusté à une concentration en albumine de 5 g/l et à un pH de 6,3 - 6,5.

Le rendement global de ces 2 étapes, chromatographie et concentration, est de l'ordre de 90 %.

Après filtration stérilisante, le produit est réparti par volumes de 5 ml, 2 ml, 1 ml ou 0,5 ml et lyophilisé, en fonction de son utilisation ultérieure sous forme de colles biologiques. La présence des stabilisants est nécessaire pour maintenir l'activité enzymatique de la thrombine au cours de ces traitements, comme le montrent les tableaux I et II.

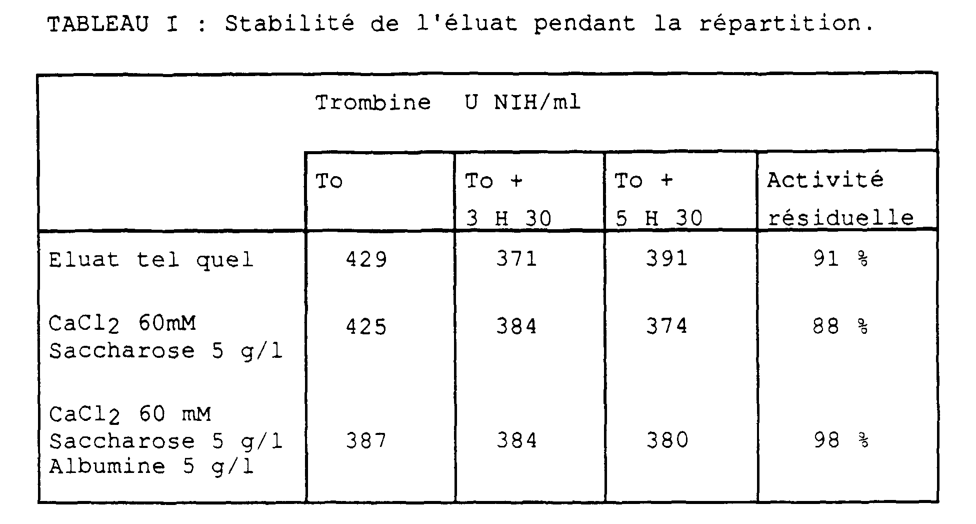

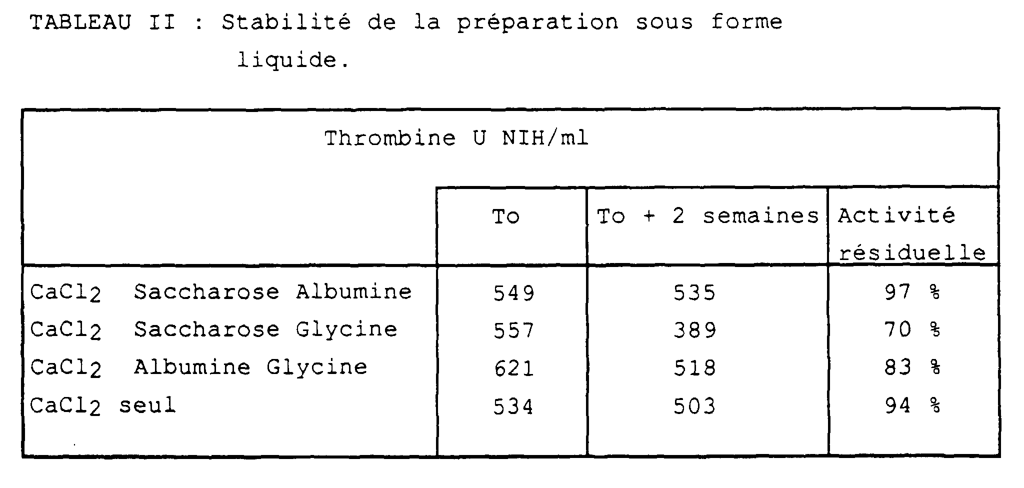

### Exemple 2 : - Caractéristiques du produit.

Le concentré de thrombine se présente sous forme lyophilisée. Après reconstitution dans l'eau distillée, il est évalué selon les méthodes suivantes :
a) - Activité coagulante.
   L'activité de l'alpha-thrombine native est mesurée par son activité coagulante sur le fibrinogène. Elle est exprimée en unités NIH (National Institute of Health) par rapport à une référence standard : thrombine du NIH, lot J.
   Après reconstitution en eau distillée, 5 dilutions de 1/300 à 1/900 sont préparées en tampon Owren Koller (L'Hémostase : méthode d'exploration et diagnostic pratique Ed. L'expansion scientifique KJ. CAEN - H.J. LARRIEU. M. SAMAMA.) contenant du polyéthylène-glycol 6000 0,5 %. La mesure du temps de coagulation est effectuée sur appareillage KC 10 Amelung (BAXTER). Dans une série de cupules, on introduit 200 µl de dilution (standard ou échantillon à tester). Après 1 minute à 37°C, on introduit 200 µl de fibrinogène à 1 g/l, en déclenchant le chronomètre. Sur papier bilogarithmique, on trace la droite standard. On reporte les temps obtenus pour les échantillons à tester et on déduit leur taux d'U NIH/ml sur le graphique.
b) - Activité amidolytique.
   L'activité amidolytique de la thrombine est dosée sur le substrat chromothrombine de STAGO selon les indications du fournisseur. Elle est exprimée en nkat/ml. La relation entre les nanokatals et les unités NIH est 1 U NIH = 2 nkat.
   L'activité trouvée sur substrat chromogénique correspond à l'activité de l'alpha-thrombine native ainsi qu'à celle de ses dérivés de protéolyse : β et γ-thrombines.
c) - Taux de protéines.
   Le taux de protéines est déterminé par la méthode au Biuret.
d) - Electrophorèse sur gel de polyacrylamide.
   Les électrophorèses sont réalisées sur le PHAST SYSTEM^{(M)} de Pharmacia en Phast gel 10/15 en milieu SDS.
e) - Propriétés de la colle biologique formée par la coagulation du concentré de fibrinogène, de Facteur XIII et de fibronectine en présence de thrombine (brevet européen 0 305 243).
   Les propriétés de la colle biologique formée en présence de thrombine ont été évaluées par les paramètres suivants : vitesse de prise en masse (secondes), pouvoir adhésif sur souris (g/cm²). Le temps de déplacement 50 (secondes) et le déplacement total (radian) ont été déterminés sur rhéomètre.

### Caractéristiques du concentré de thrombine.

Les caractéristiques mesurées selon les méthodes décrites plus haut sont reportées dans le tableau suivant.

**TABLEAU III :**

| Caractéristiques des concentrés de thrombine humaine | | | | |
|---|---|---|---|---|
| Numéro de lot | 06510010 | 06510020 | 06510031 | 06510032 |
| Volume de reconstitution (ml) | 5,2 | 2,2 | 1,2 | 5,2 |
| | | | | |
| Thrombine coagulante U NIH/ml | 610 | 540 | 564 | 522 |
| | | | | |
| Thrombine amidolytique U NIH/ml | 688 | 587 | 764 | 739 |
| | | | | |
| Taux de protéines g/l | 8 | 7 | 7 | 6,5 |
| | | | | |
| pH | 6,25 | 6,27 | 6,30 | 6,41 |
| | | | | |
| Taux de calcium mmole/l | 55 | 56 | 60 | 75 |
| | | | | |
| Osmolarité mOsm/l | 594 | 539 | 554 | 570 |
| | | | | |
| Saccharose g/l | 2,25 | 4,8 | 5,1 | 4,3 |
| | | | | |
| Albumine g/l | 6,6 | 6,4 | 6,2 | 6,0 |
| | | | | |
| Vitesse de prise en masse (sec) | 6 | 6 | 5 | 7 |
| | | | | |
| Temps de déplacement 50 (sec) | 0,77 | 0,57 | 0,81 | 0,58 |
| | | | | |
| Déplacement total (Rad) | 0,60 | 0,36 | 0,60 | 0,34 |
| | | | | |
| Pouvoir adhésif g/cm² | 130 | 160 | 137 | 142 |

L'activité coagulante dans le concentré est de l'ordre de 550 U NIH/ml.

L'activité amidolytique est du même ordre 600 - 700 U NIH/ml, démontrant l'absence d'un taux élevé de formes dégradées β et γ-thrombines.

La pureté du produit est évaluée par électrophorèse sur gel de polyacrylamide. En milieu SDS, on met en évidence, outre la bande correspondant à l'albumine de masse moléculaire 67 000, une bande majeure unique correspondant à l'α-thrombine de masse moléculaire 36 000. Après réduction par le β-mercapto-éthanol apparaît une bande correspondant à la chaîne lourde de l'α-thrombine, de masse moléculaire plus faible. La chaîne légère d'environ 4 000 daltons a migré hors de la plaque.

La stabilité du produit après reconstitution a été testée (tableau IV). La thrombine est stable au moins 24 heures à l'état liquide. Elle est également stable sous forme congelée (tableau V) ainsi que sous forme lyophilisée (tableau VI). Cette stabilité est assurée par le milieu gluconate - saccharose - chlorure de calcium - albumine, à pH 6,5.

**TABLEAU IV :**

| Stabilité de la thrombine après reconstitution | | |
|---|---|---|
| | Activité coagulante (U NIH/ml) | |
| lot n° : | 0651010 5 ml | 06510020 2 ml |
| To | 569 | 595 |
| | | |
| To + 3 H | 590 | 614 |
| | | |
| To + 6 H | 596 | 619 |
| | | |
| To + 24 H | 539 | 545 |

**TABLEAU V :**

| Stabilité de la thrombine sous forme congelée | | | |
|---|---|---|---|
| lot n° : | Activité coagulante (U NIH/ml) | | |
| | 06510020 | 06510031 | 06510032 |
| To | 610 | 598 | 595 |
| | | | |
| T + 1 mois | 611 | 628 | 582 |

**TABLEAU VI :**

| Stabilité de la thrombine sous forme lyophilisée à +4°C | | | | | |
|---|---|---|---|---|---|
| Lot 06510010 | Activité coagulante (U NIH/ml) | Vitesse de prise en masse (sec) | Temps de déplacement 50 (sec) | Déplacement total (rd) | Pouvoir adhésif (g/cm²) |
| To | 610 | 6 | 0,77 | 0,60 | 130 |
| | | | | | |
| To + 1 mois | 619 | 6 | 0,63 | 0,44 | / |
| | | | | | |
| To + 2 mois | 609 | 7 | 0,62 | 0,39 | 155 |
| | | | | | |
| To + 3 mois | 573 | 5 | 0,56 | 0,29 | 135 |
| | | | | | |
| To + 12 mois | 584 | 5 | 0,56 | 0,30 | 143 |

La stabilité de la thrombine lyophilisée a également été mesurée dans un test de "vieillissement accéléré" (Tableau VII).

**TABLEAU VII :**

| Stabilité de la thrombine sous forme lyophilisée (5 ml) à 45°C. | | | | | |
|---|---|---|---|---|---|
| Lot 06510010 | Thrombine coagulante U NIH/ml | Vitesse de prise en masse (sec) | Temps de déplacement 50 (sec) | Déplacement total (rd) | Pouvoir adhésif g/cm |
| To | 610 | 6 | 0,77 | 0,60 | 130 |
| To + 3 semaines | 508 | 6 | 0,65 | 0,43 | 151 |
| To + 6 semaines | 520 | 8 | 0,83 | 0,70 | 152 |
| To + 10 semaines | 580 | 3 | 0,45 | 0,20 | 180 |
| To + 3 mois | 555 | 5 | 0,71 | 0,44 | |

Les propriétés rhéologiques de la colle biologique obtenue en présente de thrombine humaine ou de thrombine bovine ont été comparées, suivant des protocoles de contrôle classiques.

L'étude a porté sur le même concentré de fibrinogène utilisé en mélange avec une thrombine bovine ou humaine (06510010). Les protocoles utilisés pour chaque essai sont rigoureusement identiques. Le matériel est un rhéomètre CARRIMED® CSL 100 à contrainte imposée.

### a) - Utilisation en mode fluage.

### Mesure de la vitesse de prise

Les temps de déplacement 50, t1 et t2 (temps nécessaire pour obtenir 50 % du déplacement total) sont :

| | |
|---|---|
| t2 (thrombine bovine) | 1.8 s. |
| t1 (thrombine humaine) | 0.77 s. |

Pas de différence significative.

### Etude de l'élasticité

On note une élasticité instantanée identique pour les deux produits.

### b) Utilisation en mode oscillation.

On note un seuil de rupture d'environ 2.500 N/m₂ pour les deux produits.

Les modules élastiques G' des deux colles présentent une évolution similaire dans le temps.

Le module élastique G' en fonction de la fréquence est comparable pour les deux produits.

Ces résultats confirment les observations faites à l'utilisation du produit en ce qui concerne l'aspect du caillot, l'exsudation et la vitesse de prise en masse.

L'ensemble des résultats permet de conclure que les caractéristiques rhéologiques et les propriétés mécaniques de la colle obtenue en présence de la thrombine humaine sont identiques à celles obtenues en présence de la thrombine bovine qui est le constituant actuel des colles biologiques couramment utilisées.

### Exemple 3 : Adaptation du procédé à grande échelle.

Le procédé tel que décrit dans l'exemple 1 a été appliqué à plusieurs lots de PPSB compris entre 10 et 15 litres, avec la même efficacité, comme l'indique le tableau VIII.

L'étape ultérieure de chromatographie et concentration s'effectue avec un rendement compris entre 89 et 100 %.

L'ensemble du procédé permet ainsi de produire des lots standardisés contenant plus de 10 millions d'unité NIH de thrombine.

**TABLEAU VIII :**

| Activité coagulante de la thrombine après recalcification et inactivation virale | | |
|---|---|---|
| N° de lot | Volume de PPSB traité (en ml) | Total des unités NIH produites, par lot |
| 96510040 | 10 000 | 9 276 660 |
| | | |
| 96510050 | 10 570 | 9 163 440 |
| | | |
| 96510060 | 16 310 | 10 519 950 |
| | | |
| 96510070 | 15 300 | 11 979 900 |
| | | |
| 96510080 | 14 400 | 14 976 000 |
| | | |
| 96520300 | 8 155 | 7 298 725 |
| | | |
| 96520400 | 13 250 | 16 761 250 |
| | | |
| 96520500 | 13 255 | 10 894 790 |

## Revendications

1. Procédé de préparation d'un concentré de thrombine humaine à haute activité, à usage thérapeutique, à partir d'une fraction de plasma, caractérisé en ce qu'il comprend la succession des 6 étapes suivantes :
a) l'adsorption de la fraction PPSB d'un surnageant de plasma cryoprécipité sur un gel à base de dextran greffé de groupements DEAE et le lavage de ladite fraction avec du chlorure de sodium en tampon citrate, puis l'élution du PPSB par augmentation de la concentration de chlorure de sodium ;
b) l'activation de la prothrombine en thrombine par recalcification du PPSB par l'addition de CaCl₂ à une concentration finale de 5 à 10 mM et par une incubation de courte durée à 37°C, puis une incubation d'une durée prolongée à température plus basse ;
c) un traitement d'inactivation virale par solvant-détergent du PPSB recalcifié ;
d) la purification de la thrombine par chromatographie sur gel échangeur fort de cations en milieu comprenant du gluconate de sodium, lavages et élution par addition de chlorure de sodium et de gluconate de sodium ;
e) la stabilisation de la thrombine éluée, par l'addition d'un mélange d'albumine, de saccharose et de CaCl₂ ;
f) la concentration de la solution de thrombine et sa distribution en volumes adaptés à son utilisation thérapeutique ultérieure et sa lyophilisation.

2. Procédé selon la revendication 1, caractérisé en ce que dans l'étape a), le lavage est réalisé avec du chlorure de sodium à 0,2-0,23 M en tampon citrate et que l'élution du PPSB est réalisée par augmentation de la concentration en chlorure de sodium à 0,5 M.

3. Procédé selon la revendication 1 ou 2, caractérisé en ce que dans l'étape b), la première incubation en CaCl₂ est effectuée pendant 2 heures à 37°C et la seconde pendant au moins 16 heures à 24°C.

4. Procédé selon l'une quelconque des revendications 1 à 3, caractérisé en ce que la chromatographie de l'étape d) est effectuée sur un gel d'agarose substitué par des groupements CH₂-SO₃, en milieu comprenant du gluconate de sodium 40 mM et du chlorure de sodium 0,01 M, à pH 6,5.

5. Procédé selon l'une quelconque des revendications 1 à 4, caractérisé en ce que la thrombine est éluée par une augmentation de la concentration en chlorure de sodium à 0,2 M.

6. Procédé selon l'une quelconque des revendications 1 à 5, caractérisé en ce que dans l'étape e) la thrombine éluée est stabilisée par l'addition d'un mélange comprenant 2 g/l d'albumine, 5 g/l de saccharose et du CaCl₂ 60 mM.

## Patentansprüche

1. Verfahren zur Herstellung eines Konzentrats aus menschlichem Thrombin mit hoher Aktivität zur therapeutischen Verwendung, ausgehend von einer Plasmafraktion, **dadurch gekennzeichnet,** daß es aus einer Abfolge der folgenden sechs Schritte besteht:
a) Adsorption der PPSB-Fraktion eines Überstands von Tieftemperatur-ausgefälltem Plasma auf einem Gel auf Basis von Dextran, das mit DEAE-Gruppen gepfropft ist, und Waschen dieser Fraktion mit Natriumchlorid in Citratpuffer, daraufhin Elution des PPSB durch Erhöhung der Konzentration des Natriumchlorids;
b) Aktivierung des Prothrombins zu Thrombin durch Verkalkung des PPSB durch Zugabe von CaCl₂ zu einer endgültigen Konzentration von 5 bis 10 mM und durch eine kurze Inkubation bei 37°C, daraufhin eine Inkubation über eine längere Zeitspanne bei einer tieferen Temperatur;
c) eine Behandlung zur viralen Inaktivierung durch ein Lösungsmittel-Detergenz des verkalkten PPSB;
d) Reinigung des Thrombins durch Chromatographie auf einem starken Kationenaustauschgel in einer Umgebung, die Natriumgluconat enthält, Waschen und Elution durch Zugabe von Natriumchlorid und Natriumgluconat;
e) Stabilisierung des eluierten Thrombins durch Zugabe einer Mischung aus Albumin, Saccharose und CaCl₂;
f) Konzentrierung der Thrombinlösung und ihre Verteilung auf an seine schließliche therapeutische Verwendung angepaßte Volumen und Lyophilisierung.

2. Verfahren nach Anspruch 1, **dadurch gekennzeichnet,** daß der Waschschritt in Schritt a mit 0,2 bis 0,23 M Natriumchlorid in Citratpuffer realisiert wird und daß die Elution von PPSB durch Erhöhung der Natriumchlorid-Konzentration auf 0,5 M durchgeführt wird.

3. Verfahren nach Anspruch 1 oder 2, **dadurch gekennzeichnet,** daß die erste Inkubation mit CaCl₂ in Schritt b für 2 Stunden bei 37°C bewirkt wird und daß die zweite für mindestens 16 Stunden bei 24°C durchgeführt wird.

4. Verfahren nach einem oder mehreren der Ansprüche 1 bis 3, **dadurch gekennzeichnet,** daß die Chromatographie in Schritt d auf einem Agarosegel durchgeführt wird, das mit CH₂-SO₃-Gruppen substituiert ist, in einer Umgebung, die Natriumgluconat in einer Konzentration von 40 mM und Natriumchlorid in einer Konzentration von 0,01 M bei einem pH von 6,5 enthält.

5. Verfahren nach einem oder mehreren der Ansprüche 1 bis 4, **dadurch gekennzeichnet,** daß das Thrombin durch Erhöhung der Natriumchlorid-Konzentration auf 0,2 M durchgeführt wird.

6. Verfahren nach einem oder mehreren der Ansprüche 1 bis 5, **dadurch gekennzeichnet,** daß das eluierte Thrombin in Schritt e durch Zugabe einer Mischung stabilisiert wird, die 2 g/l Albumin, 5 g/l Saccharose und 60 mM CaCl₂ enthält.

## Claims

1. A process for preparing a human thrombin concentrate with high activity, for therapeutic use, starting from a plasma fraction, characterized in that it includes the succession of the 6 following steps :
a) adsorption of the PPSB fraction of a supernatant of cryoprecipitated plasma on a dextran gel grafted with DEAE groups and washing of said fraction with sodium chloride in citrate buffer, followed by elution of PPSB by increasing the sodium chloride concentration;
b) activation of prothrombin into thrombin by recalcification of the PPSB by adding CaCl₂ at a final concentration of 5 to 10 mM, for a short period of incubation at 37°C followed by a longer period of incubation at a lower temperature;
c) a viral inactivation treatment with solvent-detergent of the recalcified PPSB;
d) purification of thrombin by chromatography on a strong cation exchanger gel in a medium containing sodium gluconate, washing and elution by adding sodium chloride and sodium gluconate;
e) stabilization of the eluted thrombin by the addition of a mixture of albumin, saccharose and CaCl₂;
f) concentration of the thrombin solution and aliquoting in volumes adapted to its subsequent therapeutic use, and freeze-drying.

2. A process according to claim 1, characterized in that in step a) washing is performed with 0,2-0,23 M sodium chloride in citrate buffer and PPSB elution is performed by increasing the sodium chloride concentration to 0,5 M.

3. A process according to claim 1 or 2, characterized in that in step b), the first incubation in the presence of CaCl₂ is performed during 2 hours at 37°C and the second one during at least 16 hours at 24°C.

4. A process according to any of claims 1 to 3, characterized in that the chromatography of step d) is performed on an agarose gel grafted with CH₂-SO₃ groups in a medium containing 40 mM sodium gluconate and 0,01 M sodium chloride at pH 6,5.

5. A process according to any of claims 1 to 4, characterized in that thrombin is eluted by increasing the sodium chloride concentration to 0,2 M.

6. A process according to any of claims 1 to 5, characterized in that in step e) the eluted thrombin is stabilized by adding a mixture of 2 g/l albumin, 5 g/l saccharose and 60 mM CaCl₂.
